Europäisches Patentamt

**European Patent Office**

**Office européen des brevets**

(11) Numéro de publication: **0 088 679**
**A1**

# DEMANDE DE BREVET EUROPEEN

(21) Numéro de dépôt: **83400417.8**

(22) Date de dépôt: **01.03.83**

(51) Int. Cl.³: **G 01 N 35/06**, G 01 N 33/04

(30) Priorité: **05.03.82 FR 8203746**

(43) Date de publication de la demande: **14.09.83**
**Bulletin 83/37**

(84) Etats contractants désignés: **AT BE CH DE GB IT LI NL SE**

(71) Demandeur: **Union de Coopératives Agricoles ENSEMBLE COOPERATIF LAITIER ELNOR, F-60490 Ressons-sur-Matz (FR)**

(72) Inventeur: **Perroud, Gérard Jean Léonard, 47, rue du Champ l'Heuillet, F-60490 Ressons-sur-Matz (FR)**

(74) Mandataire: **Durand, Yves Armand Louis et al, Cabinet Z. Weinstein 20, Avenue de Friedland, F-75008 Paris (FR)**

(54) **Procédé et machine automatique de contrôle de la qualité bactériologique d'un liquide contenu dans un récipient.**

(57) La présente invention concerne un procédé et une machine automatique de contrôle de la qualité bactériologique d'un liquide contenu dans un récipient fermé.

La machine de l'invention comprend essentiellement un convoyeur (2) à barres (8) pour acheminer pas à pas des rangées de récipients (7) à contrôler, ce convoyeur étant monté horizontalement sur un bâti (1) qui porte un plateau (3) disposé au-dessus du convoyeur (2) et supportant: une table mobile (4) recevant une platine (11) de microtitration portant des coupelles (12) contenant un réactif approprié, un dispositif (5) de prélèvement par aiguilles (17) d'une quantité minime de liquide dans les récipients (7), et un dispositif (6) de transpercement de part en part des récipients (7), le liquide étant évacué hors de la machine par l'intermédiaire d'un bec de vidange (24).

La machine de l'invention s'applique notamment au contrôle de la qualité bactériologique du lait longue conservation.

1

<u>Procédé et machine automatique de contrôle de la qualité</u>
<u>bactériologique d'un liquide contenu dans un récipient.</u>

La présente invention a essentiellement pour objet
un procédé de contrôle en continu de la qualité bactériologique d'un liquide alimentaire, tel que par exemple du lait U.H.T. ou longue conservation contenu dans
un récipient fermé en carton ou toute autre matière
appropriée.

Elle vise également une machine automatique pour la
mise en oeuvre de ce procédé.

On sait que, sur les chaînes permettant le conditionnement de denrées alimentaires liquides, en boîtes,
cartons, briks, ou analogues, il est nécessaire d'effectuer de temps en temps des prélèvements pour vérifier
tant l'emballage que les qualités organoleptiques et
bactériologiques de la denrée liquide.

C'est ainsi que pour contrôler par exemple les qualités
bactériologiques du lait conditionné dans des récipients
en cartons ou briks, il était connu de pratiquer une
découpe dans le récipient pour pouvoir y prélever,
à l'aide d'une seringue par exemple, une certaine
quantité de produit que l'on introduisait ensuite dans
un tube à essai contenant un réactif approprié et qui
était incubé plus ou moins longtemps de façon à provoquer la réaction permettant d'identifier les qualités du lait. Il s'agissait là d'une méthode quelque peu artisanale et qui, comme on le comprend, exigeait des opérations de manipulation, de nettoyage et de stérilisation des tubes. Par ailleurs, cette méthode nécessitait l'utilisation d'une quantité importante de lait, de sorte que, au total, elle demeurait coûteuse, longue à effectuer et procurait des résultats parfois aléatoires.

La présente invention a pour but de remédier notamment aux inconvénients ci-dessus, en proposant un procédé de contrôle automatisé des propriétés bactériologiques d'une denrée liquide contenue dans un récipient, ce procédé, ainsi que la machine pour sa mise en oeuvre, permettant de réaliser des tests avec une grande fiabilité et rapidité de résultat, lesquels tests sont d'une exécution peu coûteuse et n'exigent qu'une quantité très minime d'échantillon à analyser.

A cet effet, l'invention a pour objet un procédé de contrôle en continu de la qualité bactériologique d'un liquide, tel que par exemple du lait longue conservation contenu dans des récipients fermés, caractérisé en ce qu'au fur et à mesure de l'avancement pas à pas des récipients, on les perfore pour y prélever par aspiration une petite quantité de liquide qui est injectée dans des coupelles ou analogues contenant un réactif de contrôle de la qualité bactériologique dudit liquide.

On comprend donc déjà que les récipients prélevés sur la chaîne de conditionnement seront soumis à un test automatisé et permettant par conséquent d'obtenir rapidement des résultats, et ce, avec le minimum d'opérations manuelles.

Suivant une autre caractéristique de ce procédé, après le prélèvement par aspiration d'un échantillon de liqui-

3

de dans le récipient, on transperce de part en part
le récipient, et on récupère le liquide évacué.

Dès lors, on peut avantageusement, à la suite de cette
opération, vérifier ou examiner visuellement l'emballage et également récupérer le produit qui n'a pas
servi à l'analyse, ce qui évite toute perte de ce côté
là.

Selon encore une autre caractéristique du procédé de
l'invention, on effectue le prélèvement précité sur
une rangée de récipients pour injecter le liquide dans
une rangée de coupelles que l'on déplace par la suite
pour permettre l'injection du liquide prélevé dans
la rangée suivante de récipients, dans une autre rangée
de coupelles.

On voit donc que le procédé de l'invention permet de
réaliser des tests à l'échelle industrielle, c'est-à-
dire simultanément sur une pluralité de récipients.

On ajoutera encore ici, qu'avant l'injection du liquide
dans les coupelles, on effectue au moins un rinçage
de l'installation de prélèvement-injection avec le
liquide contenu dans les récipients à contrôler, ce
qui évite avantageusement tout résultat faussé en
raison de la contamination possible d'un récipient
qui ne doit évidemment pas être transmise au récipient
suivant à contrôler.

L'invention vise encore une machine automatique pour
la mise en oeuvre du procédé répondant aux caractéristiques susmentionnées, cette machine étant essentiellement caractérisée par un convoyeur à barres pour
acheminer les récipients à contrôler, ledit convoyeur
étant monté horizontalement sur un bâti qui porte un
plateau disposé au-dessus du convoyeur et supportant :

4

une table mobile recevant les coupelles précitées, un dispositif de prélèvement de liquide dans les récipients et un dispositif de percement ou tranchage de part en part de ces récipients.

Suivant une autre caractéristique de cette machine, la table mobile précitée, actionnable par vérin par exemple, est pourvue d'une platine de microtitration recevant les coupelles, ainsi que d'une goulotte d'évacuation du liquide prélevé pour rinçage.

Cette machine est encore caractérisée en ce que le dispositif précité de prélèvement comprend un groupe d'aiguilles creuses actionnées par vérin ou tout autre moyen approprié, et reliées à des becs, buses ou analogues d'injection au-dessus des coupelles, par l'intermédiaire d'une pompe péristaltique.

On notera encore ici que le dispositif de percement ou de tranchage précité est constitué par une pluralité de tranchets, poinçons ou analogues actionnables par un vérin qui, comme le vérin cité précédemment et actionnant les aiguilles, est solidaire d'un portique monté sur le bâti de la machine.

La machine selon l'invention est encore caractérisée en ce qu'à l'aplomb du dispositif précité de percement et en dessous du brin supérieur du convoyeur à barres, est prévu un bac de réception du liquide qui est évacué hors de la machine par un moyen de vidange approprié, tel que par exemple un simple bec déverseur.

On ajoutera encore ici que les coupelles précitées contiennent un réactif connu sous la dénomination "Résazurine", lequel réactif permet un contrôle fiable et précis du lait U.H.T. ou longue conservation.

5

D'autres caractéristiques et avantages du procédé et de la machine selon l'invention apparaîtront mieux dans la description détaillée qui suit et se réfère aux dessins annexés, donnés uniquement à titre d'exemple, et dans lesquels :

La figure 1 est une vue schématique et en élévation , avec arrachements, d'une machine conforme à l'invention.

La figure 2 est une vue schématique de dessus de cette machine.

La figure 3 est une vue très schématique et en bout de la machine visible sur la figure 1, et

La figure 4 est une vue partielle, de dessus, et à grande échelle, de la table portant la platine de microtitration.

Suivant un exemple de réalisation, et en se reportant aux dessins annexés, une machine conforme à l'invention pour le contrôle de la qualité bactériologique d'un liquide, tel que du lait U.H.T. ou longue conservation, contenu dans un récipient fermé, comprend essentiellement un bâti 1 supportant un convoyeur horizontal à barres 2 ainsi qu'un plateau ou analogue 3 disposé au-dessus du convoyeur 2 et supportant les éléments essentiels suivants : une table 4 mobile transversalement à la direction longitudinale du convoyeur 2, un dispositif 5 de prélèvement de liquide dans les récipients à analyser, et un dispositif 6 de percement ou de tranchage de part en part des récipients.

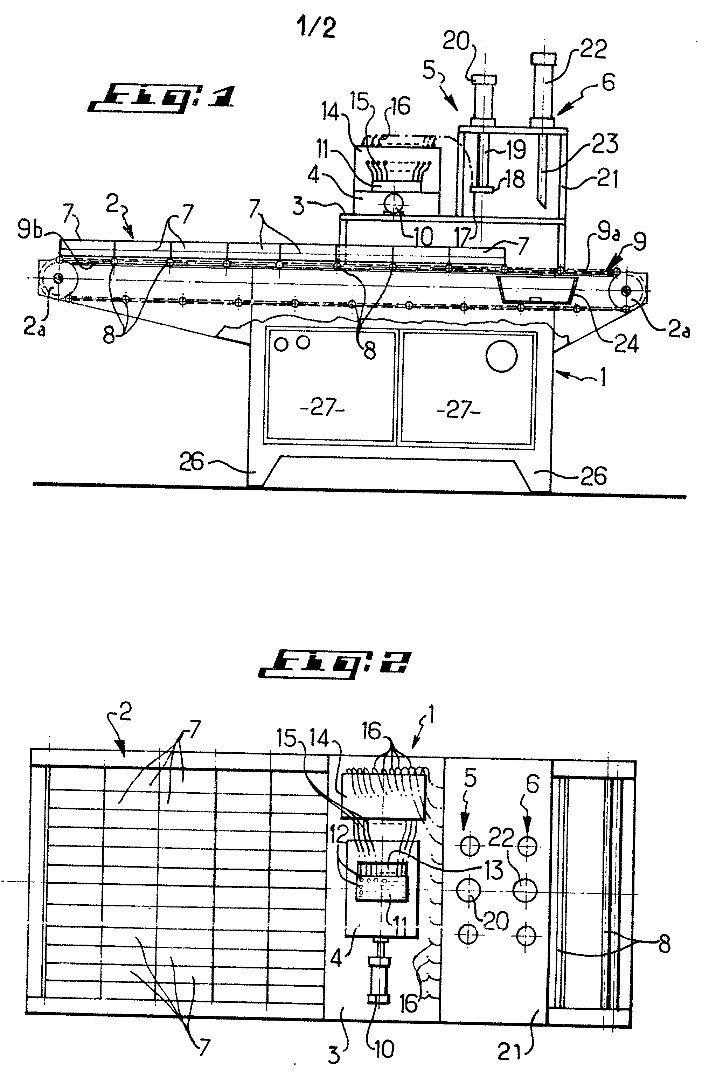

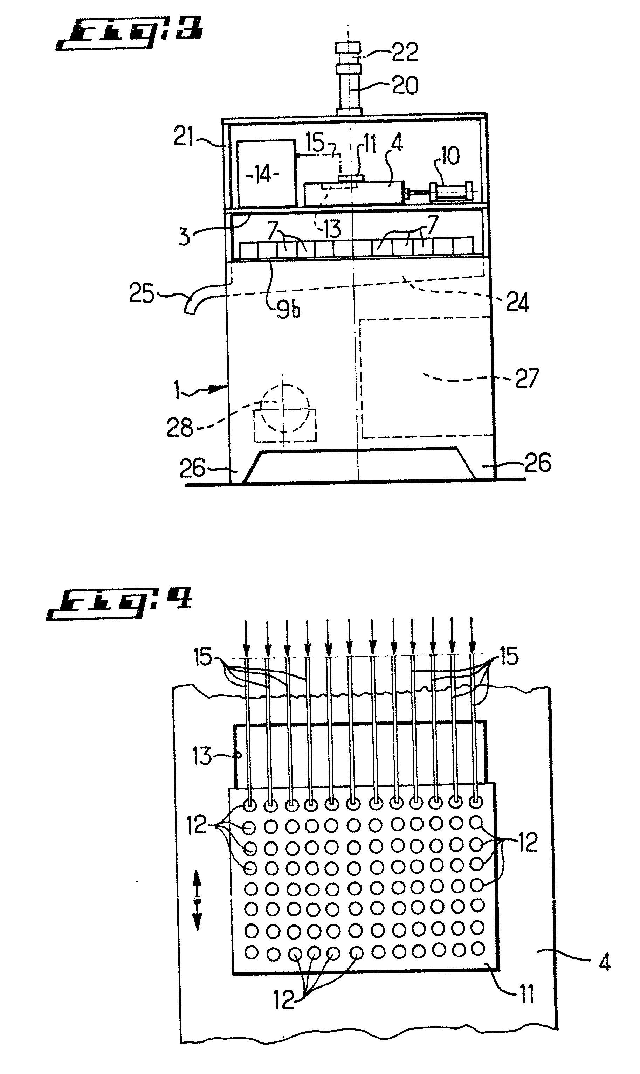

Suivant l'exemple représenté, les récipients 7 présentent, comme cela est bien connu, la forme de "briques" parallélépipédiques, lesquelles sont disposées côte à côte pour former des rangées entre les barres 8 du convoyeur 2. Les barres 8 sont montées transversalement entre deux chaînes sans fin 9 passant autour de couronnes dentées 2a et formant le convoyeur 2, comme on le voit bien sur la figure 1. Les récipients ou briques 7 reposent par leurs tranches sur une plaque de glissement 9b, bien visible sur la figure 3, et s'étendant entre les brins supérieurs 9a des chaînes 9.

Le convoyeur 2 achemine successivement les rangées de récipients ou briques 7 sous le plateau 3 qui porte la table mobile 4 ainsi que les dispositifs de prélèvement 5 de liquide dans les récipients 7, et de percement 6 de ces récipients, que l'on décrira maintenant en détail.

La table mobile 4 est actionnée par tout moyen approprié, tel que par exemple un vérin 10 et supporte une platine de microtitration 11 déplaçable par rapport à la table 4 et portant des rangées de coupelles ou analogues 12, par exemple huit rangées de douze coupelles chacune, comme on le voit mieux sur la figure 4. Dans la table mobile 4 est ménagée une goulotte 13 s'étendant en-dessous de la platine de microtitration 11 et permettant l'évacuation du lait prélevé dans les récipients 7 pour le rinçage de l'installation, comme on l'expliquera ultérieurement.

Une pompe péristaltique 14 montée sur le plateau 3 permet l'injection d'une très petite quantité de lait prélevée dans les récipients 7, dans les coupelles 12 qui contiennent une dose de réactif approprié tel que par exemple le réactif connu sous le nom de "Resazurine" et dont la couleur peut virer en fonction des qualités bactériologiques du lait analysé. Plus précisément, la sortie de la pompe 14 communique avec les coupelles 12 par une pluralité de conduites, becs ou analogues 15 débouchant au-dessus d'une rangée de coupelles 12 sur la platine 11, comme on le voit bien sur la figure 4.

D'un autre côté, la pompe péristaltique 14 communique par des tuyaux souples par exemple 16, avec autant d'aiguilles creuses et verticalement mobiles 17. Ces aiguilles sont par exemple au nombre de douze et sont solidaires d'une embase 18, formant en quelque sorte et par exemple une rampe d'aiguilles 17, ladite embase étant fixée sur la tige 19 d'un vérin 20 dont le corps est solidaire d'un portique ou analogue 21 monté sur le bâti 1 de la machine. Les aiguilles 17 sont bien entendu aptes à percer les récipients 7 d'une rangée de récipients entre deux barres 8 du convoyeur 2, pour permettre le prélèvement d'une quantité minime de liquide ou lait contenu dans ces récipients afin d'injecter ladite quantité par la pompe 14 dans les coupelles 12.

Au portique 21 du bâti 1 est également associé un vérin 22 actionnant des tranchets, poinçons ou analogues 23 susceptibles de traverser de part en part les récipients 7 après qu'un prélèvement ait été effectué par les aiguilles 17. Au dessous des tranchets 23 et du brin supérieur 9a du convoyeur 2, le bâti 1 comporte un bac de réception du liquide qui est évacué par tout moyen de vidange approprié tel que par exemple un simple bec déverseur 25 visible sur la figure 3.

Le bâti 1 comporte un socle ou des pieds 26 par l'intermédiaire desquels la machine repose sur le sol ainsi que des espèces de rangement 27. On a montré en 28, dans le bâti 1, un groupe moto-réducteur qui commande en synchronisme l'avancement pas à pas du convoyeur 2 ainsi que les vérins 10, 20 et 22 commandant respectivement le déplacement de la table mobile 4 et le déplacement vertical des aiguilles 17 et des organes de découpage et de poinçonnage 23.

8

Le fonctionnement de la machine se déduit de la description qui précède et sera brièvement décrit ci-après.

Le convoyeur 2 achemine pas à pas des rangées de douze récipients ou briques 7 contenant du lait U.H.T. ou longue conservation jusqu'au poste de prélèvement 5. A ce stade, la tige 19 du vérin 20 s'abaisse et les douze récipients de la première rangée sont percés par les aiguilles 17 qui permettent, grâce à la pompe 14, d'aspirer une quantité minime de lait qui est injectée par les buses ou becs 15 dans la première rangée de douze coupelles 12 sur la plaque de microtitration 11, comme on le voit sur la figure 4.

Ensuite, la première rangée de briques 7 passe au poste de transpercement 6, tandis que la rangée suivante de briques 7 parvient sous les aiguilles 17 pour y subir un prélèvement comme indiqué précédemment, le liquide prélevé étant envoyé par la pompe 14 dans la deuxième rangée de coupelles 12 sur la platine 11 qui a été déplacée préalablement par le vérin 10 agissant sur la table 4 portant ladite platine.

Revenant à la première rangée de briques 7, celle-ci a été transpercée par les organes de découpe ou de poinçonnage 23 et le lait qui y est contenu est recueilli dans le bac 24 puis évacué par le bec 25 qui permet de récupérer le lait dans des bidons par exemple ou bien de le recycler selon la manière désirée. Les récipients vides de la première rangée peuvent être ainsi examinés ou vérifiés visuellement.

On notera ici que la pompe 14 permet avantageusement d'effectuer deux prélèvements consécutifs dans une rangée de récipients 7 afin de rincer deux fois le circuit 17, 16, 15 avant d'injecter le lait dans les coupelles 12. Le lait de rinçage sera évacué par la goulotte 13, et ce en agissant sur la table mobile 14, de façon que les buses ou becs 15 débouchent dans ladite goulotte. Autrement dit, c'est le troisième prélèvement qui servira à l'analyse, c'est-à-dire qu'on enverra une quantité très petite de lait (environ 200 microlitres) dans chaque coupelle, l'opération étant réalisée sur douze échantillons simultanément. Cela permet de remplir en une seule fois une rangée de douze coupelles et, lorsqu'on aura rempli les huit rangées de douze coupelles correspondant respectivement à huit rangées de briques 7, on pourra alors disposer sur la table mobile 4 une autre série de coupelles 12.

L'appréciation de l'existence éventuelle de bactéries dans le lait se fera par l'observation visuelle des différentes couleurs de la résazurine dans les coupelles 12, et ce au bout d'un temps relativement court. On notera encore que les briques 7 sont positionnées sur chant longitudinal entre les barres 8 du convoyeur 2, un tel positionnement permettant d'effectuer la perforation par les aiguilles 17 en respectant la soudure longitudinale que comportent généralement de tels récipients. Enfin, on ajoutera ici que, lorsque la platine de microtitration 11 comporte 96 échantillons, on la place dans une étuve bactériologique très bien ventilée dont la température est réglée à 37°C, de sorte qu'on peut observer, après 4 heures d'étude, s'il y a eu un changement de couleur correspondant à la présence de germes éventuels dans l'échantillon.

On a donc réalisé suivant l'invention un procédé et une machine automatique de contrôle de la qualité bactériologique du lait longue conservation, ce procédé et cette machine étant entièrement automatisés ce qui réduit considérablement le coût d'analyse, et ce qui permet d'obtenir des temps d'analyse beaucoup plus courts et des résultats beaucoup plus fiables qu'auparavant, ainsi qu'un stockage beaucoup moins long et important des récipients ou briques prélevés de la chaîne de fabrication pour réaliser les tests.

11

Revendications

1. Procédé de contrôle en continu de la qualité bactériologique d'un liquide, tel que par exemple du lait
longue conservation, contenu dans des récipients fermés,
et du type consistant à perforer ces récipients pour
y prélever par aspiration une petite quantité de liquide
qui est injectée respectivement dans des coupelles ou
analogues contenant un réactif de contrôle de la qualité
bactériologique dudit liquide, caractérisé en ce
qu'avant l'injection du liquide dans les coupelles on
effectue au moins un rinçage du circuit de prélèvement-
injection à l'aide du liquide contenu et prélevé dans
les récipients à contrôler, tandis qu'après ledit prélèvement on transperce de part en part lesdits récipients
pour les vérifier et on récupère le liquide évacué.

2. Machine automatique pour la mise en oeuvre du procédé
selon la revendication 1 et du type comprenant un bâti
supportant un dispositif de prélèvement du liquide contenu dans les récipients ainsi qu'une table mobile recevant les coupelles précitées au-dessus desquelles sont
prévus des becs ou buses d'injection reliés au dispositif de prélèvement, caractérisée par un dispositif (6)
de percement ou tranchage de part en part des récipients
(7), ainsi que par une platine de microtitration (11)
recevant lesdites coupelles (12) et déplaçable sur ladite table mobile (4) qui est pourvue d'une goulotte (13)
d'évacuation du liquide prélevé pour le rinçage.

3. Machine selon la revendication 2, caractérisée en
ce que le dispositif de percement précité (6) est constitué par une pluralité de tranchets ou poinçons (23)
actionnables par un vérin (22) solidaire d'un portique
(21) monté sur le bâti (1) de la machine.

4. Machine selon la revendication 2 ou 3, et dans laquelle le dispositif de prélèvement est constitué par un groupe d'aiguilles creuses, caractérisée en ce que lesdites aiguilles (17) sont actionnées par un vérin (20) solidaire du portique précité (21).

5. Machine selon l'une des revendications 2 à 4, caractérisée en ce qu'à l'aplomb du dispositif précité de percement (6) et en dessous du brin supérieur (9a) d'un convoyeur à barres (2) pour l'acheminement pas à pas des récipients (7), est prévu un bac (24) de réception du liquide qui est évacué hors de la machine par un moyen de vidange approprié tel que par exemple un bec déverseur (25).

6. Machine selon l'une des revendications 2 à 5, caractérisée en ce que les récipients précités (7) sont constitués par des boîtes ou briks contenant du lait dont le prélèvement et l'injection dans les coupelles (12) sont assurés par une pompe péristaltique (14).

Fig. 1

Fig. 2

**Fig:3**

**Fig:4**

0088679

Office européen
des brevets

**RAPPORT DE RECHERCHE EUROPEENNE**

Numéro de la demande

EP 83 40 0417

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | FR-A-2 317 634 (DYNATECH LABORATORIES INC.) * Page 1, ligne 1 - page 2, ligne 10; figures 2,4 * | 1,4 | G 01 N 35/06 G 01 N 33/04 |
| Y | FR-A-1 243 755 (A. SCHWARTE) * Page 3, colonne de gauche, lignes 20-26; figure 1 * | 1 | |
| A | FR-A-2 361 655 (SVENSKA MEJERIERNAS RIKSFORENINGS EKONOMI AB.) * Page 1, lignes 1-18; page 3, lignes 13-17; page 4, lignes 33-35; figure 1 * | 1 | |
| A | EP-A-0 018 092 (LILLY INDUSTRIES LTD.) * Page 5, ligne 35 - page 6, ligne 12; figure 1 * | 1,4,6 | DOMAINES TECHNIQUES RECHERCHES (Int. Cl. 3) |
| A | US-A-3 683 977 (J.A. CROWE et al.) * Colonne 1, lignes 59-65; colonne 2, lignes 56-67; colonne 3, lignes 38-44; figure 1 * | 1,3,4 | G 01 N 1/00 G 01 N 35/00 G 01 N 33/00 |
| A | FR-A-2 417 093 (SOCIETE FRANCAISE POUR LE DEVELOPPEMENT DE L'AUTOMATISME EN BIOLOGIE) * Page 6, lignes 1-21; figures 1,4 * | 1,4 | |

--- -/-

Le présent rapport de recherche a été établi pour toutes les revendications

| Lieu de la recherche LA HAYE | Date d'achèvement de la recherche 15-06-1983 | Examinateur ANTHONY R.G. |
|---|---|---|

Office européen
des brevets

**RAPPORT DE RECHERCHE EUROPEENNE**

| | **DOCUMENTS CONSIDERES COMME PERTINENTS** | | **Page 2** |

| Catégorie | Citation du document avec indication, en cas de besoin, des parties pertinentes | Revendication concernée | CLASSEMENT DE LA DEMANDE (Int. Cl. ³) |
|---|---|---|---|
| A | FR-A-1 104 791 (R.L.J. SIMONIN et al.) ----- | | |

**DOMAINES TECHNIQUES RECHERCHES (Int. Cl. ³)**

Le présent rapport de recherche a été établi pour toutes les revendications

| Lieu de la recherche LA HAYE | Date d'achèvement de la recherche 15-06-1983 | Examinateur ANTHONY R.G. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03 82